(19) Europäisches Patentamt
European Patent Office
Office européen des brevets

(11) Publication number: **0 623 601 A1**

(12) **EUROPEAN PATENT APPLICATION**

(21) Application number: **94201160.2**

(22) Date of filing: **27.04.94**

(51) Int. Cl.[5]: **C07D 233/20**, C07D 233/52, C07D 239/06, C07D 239/18, C07D 277/10, C07D 277/18, C07D 207/20, C07D 207/22, A01N 43/50, A01N 43/54, A01N 43/36, A01N 43/78

(30) Priority: **30.04.93 EP 93303424**

(43) Date of publication of application: **09.11.94 Bulletin 94/45**

(84) Designated Contracting States: **AT BE CH DE DK ES FR GB GR IE IT LI LU MC NL PT SE**

(71) Applicant: **SHELL INTERNATIONALE RESEARCH MAATSCHAPPIJ B.V.**
**Carel van Bylandtlaan 30**
**NL-2596 HR Den Haag (NL)**

(72) Inventor: **Davies, Sarah Anne**
**43 Woodside Gardens**
**Sittingbourne, Kent ME10 1SG (GB)**
Inventor: **Mankee, Jamie Brian**
**66 Volante Drive,**
**Grovehurst**
**Sittingbourne, Kent ME10 2JJ (GB)**
Inventor: **Mete, Antonio**
**7 Bradley Drive**
**Sittingbourne, Kent ME10 1RB (GB)**

(74) Representative: **Allam, Peter Clerk et al**
**LLOYD WISE, TREGEAR & CO.**
**Norman House**
**105-109 Strand**
**London WC2R 0AE (GB)**

(54) **N-(3-Fluorobenzyl)heterocyclic derivatives and their use as pesticides.**

(57) Compound of general formula:

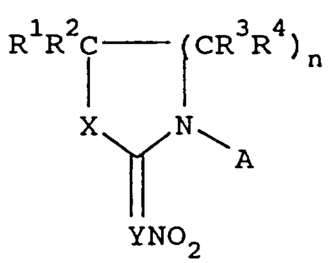

(I)

in which n is 1, 2 or 3; $R^1$, $R^2$, $R^3$ and $R^4$ independently represent a hydrogen atom or an alkyl group; X represents and oxygen atom or a group $>CR^5R^6$, $>NR^7$, $>NCOR^8$ or $>S(O)_p$, where $R^5$, $R^6$, $R^7$ and $R^8$ independently represent a hydrogen atom or an alkyl group and p is 0, 1 or 2; A represents an optionally substituted 3-fluorobenzyl group; and Y represents a group =CH or =N- are disclosed. The compounds are pesticidal.

EP 0 623 601 A1

This invention relates to certain N-benzyl heterocyclic compounds, to processes for their preparation and to their uses as biocides, for example, as pesticides.

N-benzyl heterocyclic compounds have been relatively widely investigated in the past. For example, JP 63287764 (Nihon Tokushu Noyaku) discloses 1-(3-cyanobenzyl)-2-(nitromethylene)imidazoline, 1-(3-cyanobenzyl)-2-(nitro-imino)imidazoline and 1-(3-cyanobenzyl)-2-(nitromethylene)tetrahydropyrimidine compounds. It is stated that the compounds are useful as insecticides, effective against Coleoptera, Lepidoptera, Hemiptera and Diptera.

DE OS 25 14 402 A1 (Hoechst) discloses certain 2-(nitromethylene)imidazolidine and 2-(nitromethylene)hexahydropyrimidine compounds which are stated to be insecticidal. The document specifically discloses 1-benzyl-2-(nitromethylene)imidazoline and 1-benzyl-2-(nitromethylene)hexahydropyrimidine.

DE OS 27 32 660 (Hoechst) discloses certain insecticidal 2-(nitromethylene)imidazolidine compounds. Specific examples disclosed include 1-(4-chlorobenzyl)-2-(nitromethylene)imidazolidine, 1-(3,4-dichlorobenzyl)-2(nitromethylene)imidazolidine, 1-(2-chlorobenzyl)-2-(nitromethylene)imidazolidine and 1-(4-fluorobenzyl)-2-(nitromethylene)imidazolidine.

European Patent Publication No. 136 636 (Nihon Tokushu Noyaku Seizo) discloses 2-(nitromethylene)-tetrahydropyrimidine derivatives which are stated to be insecticidal. Specfic examples disclosed include 1-(4-chlorobenzyl)-2-(nitromethylene)tetrahydropyrimidine, 1-(4-bromobenzyl)-2-(nitromethylene)-tetrahydropyrimidine and 1-(4-fluorobenzyl)-2-(nitromethylene)tetrahydropyrimidine.

This invention is based upon the discovery of interesting pesticidal activity of certain N-(3-fluorobenzyl)-heterocyclic compounds.

According to a first aspect of the present invention, there is provided a compound of general formula:

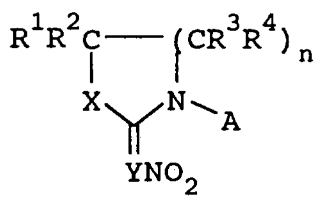

(I)

in which n is 1, 2 or 3; $R^1$, $R^2$, $R^3$ and $R^4$ independently represent a hydrogen atom or an alkyl group; X represents an oxygen atom or a group $>CR^5R^6$, $>NR^7$, $>NCOR^8$ or $>S(O)_p$, where $R^5$, $R^6$, $R^7$ and $R^8$ independently represent a hydrogen atom or an alkyl group and p is 0, 1 or 2; A represents an optionally substituted 3-fluorobenzyl group; and Y represents a group =CH- or =N-.

It has been found that the presence of a 3-fluorobenzyl group tends to lead to increased pesticidal activity of compounds of general formula I in comparison to similar compounds wherein A represents, for example, a 3-chlorobenzyl, a 3-bromobenzyl or an unsubstituted benzyl group.

Unless otherwise specified in this specification, an alkyl group may be linear or branched and suitably contains up to 10, preferably up to 6, and most preferably up to 4, carbon atoms, preferred examples being methyl and ethyl.

Unless otherwise stated in this specification, when any groups are designated as being optionally substituted, the substituent groups which are optionally present may be any of those customarily employed in the development of pesticidal compounds, and/or the modification of such compounds to influence their structure/activity, persistence, penetration or other property. In relation to an alkyl group, specific examples of such substituents may include halogen, specially fluorine, chlorine or bromine atoms, and phenyl, alkoxy, hydroxy, cyano and (alkyl)amino groups. In relation to a phenyl moiety, optional substituents may include halogen atoms, for example fluorine, chlorine, bromine and iodine atoms, and nitro, cyano, alkoxy, hydroxy, amino, (alkyl)amino, alkyl, alkylthio and haloalkyl (especially $CF_3$) groups.

Preferably, n is 1 or 2.

$R^1$, $R^2$, $R^3$ and $R^4$ may independently represent a hydrogen atom or a methyl group. Preferably, at least three of $R^1$, $R^2$, $R^3$ and $R^4$ represent a hydrogen atom. More preferably, each of $R^1$, $R^2$, $R^3$ and $R^4$ represent a hydrogen atom.

Preferably, X represents a group $>NR^7$ or $>S(O)_p$.More preferably, X represents a group $>NR^7$. Where X represents a group $>NR^7$, preferably $R^7$ represents a hydrogen atom. Where X represents a group $>S(O)_p$, preferably p is 0.

In an optionally substituted 3-fluorobenzyl group, optional substituents may be slected from halogen atoms or alkyl, amino, alkylamino (which term includes mono-, di- and tri-alkylamino), cycloamino, alkoxy,

haloalkoxy, alkylsulphyl, alkylsulphinyl, alkylsulphonyl, carboxy, alkylcarboxy, monohaloalkyl, dihaloalkyl, trihaloalkyl, nitro, phenoxy and cyano groups.

The 3-fluorobenzyl group may be unsubstituted. Preferably, it is substituted and, preferably, optional substituents of said 3-fluorobenzyl group are selected from halogen atoms, or alkyl or alkylamino groups. Preferred halogen atoms are fluorine and chlorine atoms, with a chlorine atom being more preferred. A preferred alkyl group is a methyl group. A preferred alkylamino group is a dialkylamino group, with dimethylamino and diethylamino being especially preferred.

The most preferred substituent of said 3-fluorobenzyl group is a dialkylamino group.

The 3-fluorobenzyl group may be substituted at each position of its benzene ring. Preferably, at least two positions, more preferably three positions, on said benzene ring are unsubstitued - that is, are hydrogen atoms. Preferably, said 3-fluorobenzyl group is substituted at the 4-position.

Preferably, said optionally substituted 3-fluorobenzyl group represents an unsubstituted 3-fluorobenzyl group, a (3-fluoro-4-chloro)benzyl group, a (3-fluoro-4-methyl)benzylgroup, a (3-fluoro-4-methoxy)benzyl group, a (3-fluoro-4-dimethylamino)benzyl group or a (3-fluoro-4-diethylamino)benzyl group. Most preferably, said optionally substituted 3-fluorobenzyl group represents a (3-fluoro-4-dimethylamino)benzyl group.

According to a second aspect of the present invention, there is provided a process for the preparation of a compound of general formula I, the process comprising:

(a) reacting a compound of general formula

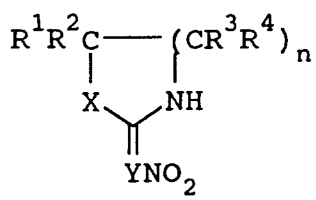

(II)

with a compound of general formula $AL^1$ or a salt thereof, wherein n, $R^1$, $R^2$, $R^3$, $R^4$, X, A and Y are as defined above and $L^1$ is a leaving group, and optionally derivatising the product of the reaction in order to prepare a compound of general formula I; or

(b) reacting a compound of general formula

$HX\text{-}CR^1R^2\text{-}(CR^3R^4)_n\text{-}NH\text{-}A$ (III)

with 1,1-bis(methylthio)-2-nitroethylene or with nitroguanidine, where n, $R^1$, $R^2$, $R^3$, $R^4$, X and A are as defined above, and optionally derivatising the product of the reaction in order to prepare a compound of general formula I.

$L^1$ preferably represents a halogen atom or a mesyl or tosyl group. Preferably $L^1$ represents a halogen atom, with a chlorine or bromine atom being especially preferred.

Reaction (a) is preferably carried out in the presence of an alkali metal hydride, for example, sodium hydride. Suitably, a compound of general formula II is dissolved in a solvent, preferably in a polar aprotic solvent, for example, in dimethylformamide, with stirring and under an inert, for example, a nitrogen atmosphere and said alkali metal hydride is then added. The compound of general formula $AL^1$ may then be added with stirring and allowed to react at ambient temperature over a period of over 10 hours. The desired product may then be isolated using standard techniques.

Compounds of general formula II for use in reaction (a) may be prepared by reacting a compound of general formula

$HX\text{-}CR^1R^2\text{-}(CR^3R^4)_n\text{-}NH_2$ (IV)

or a salt thereof, n, $R^1$, $R^2$, $R^3$, $R^4$ being as defined above, with 1,1-bis(methylthio)-2-nitroethylene or with nitroguanidine. The reaction is preferably carried out in water or an alcohol solvent, for example ethanol, under reflux.

Where X represents a group $-S(O)_p$, p is preferably 0 in said compound of general formula IV and the reaction may use a salt of said compound of general formula IV, for example a hydrochloride thereof.

Compounds of general formula IV are either known or may be made by standard procedures. 1,1-Bis-(methylthio)-2-nitroethylene and nitroguanidine are commercially available.

Compounds of general formula $AL^1$ for use in reaction (a) are, in many cases, commercially available. Where not commercially available, they may be readily prepared from appropriate 3-fluorobenzoic acid derivatives by reaction of the benzoic acid derivative with a halogenating agent, for example, thionyl chloride, under reflux. The product of the reaction may then be added to sodium borohydride in water and the reaction allowed to proceed with stirring at ambient temperature. The product may then be isolated by standard techniques, and is a benzyl alcohol. This can be converted to the benzyl halide as described below.

An alternative process for the preparation of compounds of general formual $AL^1$, for use particularly, although not exclusively, for compounds wherein an optional substituent of group A is an alkylamino group involves reacting, for example, the appropriate 3-fluoro-alkylamino benzyl alcohol with thionyl chlroide at ambient temperature. Benzyl alcohols are readily prepared from benzoic acid derivatives by reaction in the presence of a hydride, for example, lithium aluminium hydride. The desired benzoic acid derivatives may be prepared from the appropriate benzonitrile compounds. 3-Fluoro-alkylaminobenzonitrile compounds may be prepared from the appropriate difluorobenzonitrile compound by heating the latter compound in a polar aprotic solvent, for example, dimethylformamide, with potassium carbonate. Alkylamine may then be added to the reaction mixture and the desired product isolated by standard techniques. The appropriate difluorobenzonitrile compounds are either commercially available or may be prepared using standard procedures.

Compounds of general formula II where X represents a group $>NCOR^8$ as defined above may also be prepared from other compounds of general formula II where X represents >NH, by a process as described in U.K. Patent Application Number GB 2 190 674 A (Shell).

Reaction (b) is preferably carried out under reflux in the presence of a solvent, for example, an alcohol. After the reaction the desired product may be isolated by standard techniques.

Compounds of general formula III for use in reaction (b) may be prepared by reacting a compound of general formula IV as described above with a compound of general formula $AL^1$ or a salt thereof, where n, $R^1$, $R^2$, $R^3$, $R^4$ and $L^1$ are as defined above. In the process, said compound of general formua IV, in a solvent, for example, acetonitrile, is preferably cooled to less than ambient temperature prior to the addition of the compound of general formula $AL^1$. Compounds of general formula IV and $AL^1$ may be prepared as described above.

The compounds of the general formula I exhibit pesticidal, for example insecticidal and/or molluscicidal activity. Accordingly the invention also provides a pesticidal composition comprising a compound of the general formula I together with a carrier. The invention further provides a method of combating pests at a locus which comprises applying to the locus a pesticidal compound or composition according to the invention.

In the method of the invention, the locus may be an agricultural or horicultural locus, for example, plants subject to attack, seeds of such plants or the medium in which such plants are growing or are to be grown. A locus as described above may suitably be treated with a compound I at an application rate in the range of 0.005-4 kg/ha, preferably 0.01-1 kg/ha. The compounds of the general formula I may be of particular interest in combating caterpillars. The compounds also exhibit activity against aphids, rice prests, whitefly, soil insects and molluscs. The compound 1-[(3-fluoro-4-dimethylamino)benzyl]-2-(nitromethylene)-imidazoline shows particularly interesting activity.

A carrier in a composition according to the invention is any material with which the active ingredient is formulated to facilitate application to the locus to be treated, or to facilitate storage, transport or handling. A carrier may be a solid or a liquid, including a material which is normally gaseous but which has been compressed to form a liquid, any any of the carriers normally used in formulating biocidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Compositions are often formulated and transported in a concentrated form which is subsequently diluted by the user before application. The presence of small amounts of a carrier which is a surface-active agent facilitates this process of dilution. Thus, preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium slats of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing 0.5-10% w of active ingredient. Granules are usually prepared to have a size between 10 and 100 BS mesh (1.676 - 0.152 mm), and may be manufactured by agglomeration or impregnation techniques. Generally, granules will contain 0.5-75% w active ingredient and 0-10% w of additives such as stabilisers, surfactants, slow release modifiers and binding agents. The so-called "dry flowable powders" consist of relatively small granules having a relatively higher concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anitfreeze agents for water.

Aqueous dispersions and emulsions, for example compositions obtained by diluting a wettable powder or a concentrate according to the invention with water, also lie within the scope of the inventoin. The said emulsions may be of the water-in-oil or of the oil-in-water type, and may have a thick "mayonnaise"-like consistency.

The inveniton further extends to the use of a compound of general formula I as a pesticide.

The composition of the invention may also contain other ingredients, for example other compounds possessing herbicidal, insecticidal or fungicidal properties.

The invention will now be described further with reference to the following Examples.

**EXAMPLE 1**

**Preparation of 1-(3-fluoro-4-chlorobenzyl)-2-(nitromethylene) imidazoline**

1.(i) Preparation of 2-(nitromethylene)imidazoline

1,1-Bis(methylthio)-2-nitroethylene (ex. Aldrich Chemical Co.) (49.5 g) and ethane-1,2-diamine (25 ml) were heated at reflux in ethanol (250 ml) for 4 hours. The mixture was allowed to cool to 20 °C. The product crystallised out and was collected by filtration.

mpt = 168 - 169 °C.

1.(ii) Preparation of 3-fluoro-4-chlorobenzyl chloride

3-Fluoro-4-chlorobenzoic acid (ex. Yarsley Fluorochemicals Ltd) (10.5 g) and thionylchloride (40 ml) were heated at reflux for 3 hours. Excess thionyl chloride was evaporated off under reduced pressure to leave a crystalline solid. The crystalline solid was added to sodium borohydride (9.0 g) in water (50 ml) and the mixture was stirred at 20°C for 18 hours. The reaction mixture was poured into water (200 ml) and extracted with dichloromethane (3 x 70 ml). The combined dichloromethane extracts were washed with water (100 ml), saturated brine (100 ml), dried over solid magnesium sulphate and evaporated down to an oil (8.2 g). This oil was dissolved in chloroform (50 ml), treated with thionyl chloride (4.5 ml) and heated at reflux for 3 hours. The reaction mixture was evaporated to dryness under reduced pressure and the residue was extracted from water (200 ml) using dichloromethane (3 x 70 ml). The combined dichloromethane extracts were washed (water: 100 ml; brine: 100 ml) dried over magnesium sulphate and evaported to a pale yellow solid (8.03 g). This was the required product and used without further purification.

1.(iii) Preparation of 1-(3-fluoro-4-chlorobenzyl)-2-(nitromethylene)imidazoline

2-(Nitromethylene)imidazoline (2.6 g) prepared in 1(i) was dissolved in dry dimethylformamide (50 ml) at 0° C. Sodium hydride (1.0 g of a 50% oil suspension) was added and the mixture was stirred, under a nitrogen atmosphere, for 30 minutes. 3-Fluoro-4-chlorobenzyl chloride (4.0 g) prepared in 1.(ii) was added and the mixture was stirred and allowed to attain ambient temperature (20°C) over 18 hours. The mixture was poured into water (200 ml) and extracted with dichloromethane (3 x 70 ml). The combined dichloromethane extracts were washed with saturated brine (100 ml), dried over solid magnesium sulphate, filtered and evaporated to dryness under reduced pressure. The solid residue was recrystallised from ethyl acetate to give a white solid (1.14 g).

| | | C | H | N |
|---|---|---|---|---|
| Analysis: | Theory:<br>Found: | 48.6<br>48.6 | 4.1<br>4.1 | 15.5<br>15.3 |

**EXAMPLE 2**

**Preparation of 1-(3-fluoro-4-chlorobenzyl)-2-(nitromethylene)tetrahydropyrimidine**

2.(i) Preparation of 2-(nitromethylene)tetrahydropyrimidine

By using propane-1,3-diamine (25 ml) instead of ethane-1,2-diamine in the procedure of Example 1(i) above, 2-(nitromethylene)tetrahydropyrimidine (38.8 g) was prepared.
mpt = 207-208°C

| | | C | H | N |
|---|---|---|---|---|
| Analysis: | Theory:<br>Found: | 42.0<br>42.0 | 6.3<br>6.3 | 29.4<br>29.6 |

2.(ii) Preparation of 1-(3-fluoro-4-chlorobenzyl)-2-(nitromethylene)tetrahydropyrimidine

2-(Nitromethylene)tetrahydropyrimidine (2.86 g) prepared in Example 2(i) and 3-fluoro-4-chlorobenzyl chloride (4.0 g) prepared in Example 1(ii) were reacted following the procedure of Example 1(iii) to produce, on recrystallisation from isopropylalcohol, the desired product as a white solid (0.82 g).

| | | C | H | N |
|---|---|---|---|---|
| Analysis: | Theory:<br>Found: | 50.4<br>50.7 | 4.6<br>4.6 | 14.7<br>14.5 |

**EXAMPLE 3**

**Preparation of 3-(3-fluoro-4-chlorobenzyl)-2-(nitromethylene)thiazolidine**

3.(i) Preparation of 2-(nitromethylene)thiazolidine

By using 2-aminoethanethiol hydrochloride (12 g) and the procedure of Example 1(i) above, 2-(nitromethylene)thiazolidine (11.6 g) was obtained as a pale yellow solid.

3.(ii) Preparation of 3-(3-fluoro-4-chlorobenzyl)-2-(nitromethylene)thiazolidine

Starting with 2-(nitromethylene)thiazolidine (1.5 g) prepared in Example 3(i) and using the procedure described in Example 1(iii), the desired product (0.45 g) was obtained as a pale yellow solid by crystallisation from ethyl acetate.

| | | C | H | N |
|---|---|---|---|---|
| Analysis: | Theory:<br>Found: | 45.8<br>46.2 | 3.5<br>3.4 | 9.7<br>9.8 |

**EXAMPLE 4**

**Preparation of 1-[(3-fluoro-4-dimethylamino)benzyl]-2-(nitromethylene)imidazoline**

4.(i) Preparation of 3-fluoro-4-dimethylaminobenzonitrile

3,4-Difluorobenzonitrile (ex. Aldrich Chemical Co.; 10 g), and potassium carbonate (9.93 g) in DMF (50 ml) were heated at 60 °C. Gaseous dimethylamine was bubbled through the reaction mixture for 20 minutes. The mixture was then allowed to cool to 20 °C and diluted with water (100 ml). The resultant mixture was extracted with diethyl ether (3 x 100 ml). The diethyl ether extracts were washed with water (100 ml), dried over magnesium sulphate and evaporated to a white solid (11.73 g).
m.p. = 35 °C

| | | C | H | N |
|---|---|---|---|---|
| Analysis: | Theory:<br>Found: | 65.8<br>65.0 | 5.5<br>5.5 | 17.1<br>16.8 |

4.(ii) Preparation of 3-fluoro-4-dimethylamino benzoic acid

3-Fluoro-4-dimethylamino benzonitrile (14.3 g) prepared in Example 4(i) in water (80 ml) and concentrated sulphuric acid (80 ml) were heated at 110 °C for 18 hours. The solution was then cooled to 20 °C and neutralised with solid sodium hydrogen carbonate. Acidification with glacial acetic acid lead to a white solid precipitating. This was collected by filtration, dissolved in dichloromethane and the resultant solution was dried over magnesium sulphate. The dichloromethane solution was then filtered and evaporated to give the product as a beige-coloured solid (11.5 g).
m.p. = 172 °C

4.(iii) Preparation of 3-fluoro-4-dimethylamino benzyl alcohol

To an ice-cooled suspension of lithium aluminium hydride (2.38 g) in diethyl ether (100 ml), stirred under an atmosphere of nitrogen, was added portionwise, 3-fluoro-4-dimethylamino benzoic acid (11.50 g) prepared in Example 4(ii). The reaction temperature was kept below 10°C throughout the addition. After stirring for 1 hour, water (2.38 ml) was added, followed by a 10% sodium hydroxide solution (2.38 ml, aqueous) and further water (7.1 ml). A solid formed which was removed by filtration and the diethyl ether solution was evaporated to give the required product as a yellow oil (10.5 g).

| | | C | H | N |
|---|---|---|---|---|
| Analysis: | Theory:<br>Found: | 63.9<br>63.8 | 7.2<br>7.5 | 8.3<br>7.9 |

4.(iv) Preparation of 3-fluoro-4-dimethylamino benzyl chloride hydrochloride

To a solution of 3-fluoro-4-dimethylamino benzyl alcohol (2.0 g) prepared in Example 4(iii) in chloroform (50 ml) was added thionyl chloride (1 ml). An exothermic reaction occurred and when the reaction mixture had cooled down to ambient temperature it was further heated at reflux for half-an-hour. The solvent was evaporated to leave the product as orange needles, (2.62 g).

| | | C | H | N |
|---|---|---|---|---|
| Analysis: | Theory:<br>Found: | 48.2<br>48.1 | 5.4<br>5.6 | 6.2<br>6.1 |

4.(v) Preparation of N-[3-fluoro-4-dimethylamino)benzyl]ethane-1,2-diamine

Ethane-1,2-diamine (1.3 ml) in acetonitrile (100 ml) was cooled to 5°C in an ice-bath. (3-Fluoro-4-dimethylamino)benzyl chloride hydrochloride prepared in Example 4. (iv) (1.41 g) in acetonitrile (25 ml) was added dropwise over 1 hour. The mixture was allowed to attain ambient temperature (20°C) and stirred for 2 hours. The reaction mixture was filtered to remove a white precipitate. The filtrate was evaporated under reduced pressure to leave the required product which was obtained as an oil (1.19 g) and used with no further purification.

4.(vi) Preparation of 1-[(3-fluoro-4-dimethylamino)benzyl]-2-(nitromethylene)imidazoline

N-[(3-fluoro-4-dimethylamino)benzyl]ethane-1,2-diamine (1.1 g) and 1,1-bis(methylthio)-2-nitroethylene (0.86 g) in isopropyl alcohol (30 ml) were heated at reflux for 12 hours. The reaction mixture was allowed to cool to ambient temperature and a yellow solid precipitated. The solid was collected by filtration, dissolved in dichloromethane, washed with water and the dichloromethane solution was then dried over anhydrous magnesium sulphate and filtered. The solution was evaporated to dryness to afford the product as a cream-coloured solid (0.33 g).
m.p. = 124°C

| | | C | H | N |
|---|---|---|---|---|
| Analysis: | Theory:<br>Found: | 55.7<br>56.1 | 6.1<br>6.1 | 20.0<br>20.2 |

## EXAMPLE 5

### Preparation of 1-[(3-fluoro-4-dimethylamino)benzyl]-2-(nitromethylene)tetrahydropyrimidine

5.(i) Preparation of N-[(3-fluoro-4-dimethylamino)benzyl]propane-1,3-diamine

By using propane-1,3-diamine instead of ethane-1,2-diamine in the procedure of Example 4(v), the required product was obtained as an oil and used without further purification.

5.(ii) Preparation of 1-[(3-fluoro-4-dimethylamino)benzyl]-2-(nitromethylene)tetrahydropyrimidine

By using N-[(3-fluoro-4-dimethylamino)benzyl]propane-1,3-diamine (1.2 g) prepared in Example 5(i) in the procedure described in Example 4(vi), the required product was obtained as a cream-coloured solid (0.51 g).
m.p. = 149°C

| | | C | H | N |
|---|---|---|---|---|
| Analysis: | Theory:<br>Found: | 57.1<br>56.8 | 6.5<br>6.5 | 19.0<br>19.0 |

## EXAMPLE 6

### Preparation of 1-(3-fluoro-4-methoxybenzyl)-2-nitroimino-3-methyl-imidazoline

6.(i) Preparation of 3-fluoro-4-methoxybenzonitrile

Sodium metal (1.0 g) was added to methanol (50 ml) and the mixture was stirred under nitrogen gas, until complete dissolution had occurred. The solution was cooled in an ice-bath and 3,4-difluorobenzonitrile (5.0 g) was added and kept cool for 2 hours. The solvent was then removed by evaporation to leave a white solid. This was the required product and was used in Example 6. (ii), without further purification.

6.(ii) Preparation of 3-fluoro-4-methoxybenzoic acid

The product was obtained by the hydrolysis of 3-fluoro-4-methoxybenzonitrile, using the procedure described in Example 4. (ii).
m.p. = 213°C

6.(iii) Preparation of 3-fluoro-4-methoxybenzyl alcohol

The required product was obtained as a yellow oil, by reduction of 3-fluoro-4-methoxybenzoic acid prepared in Example 6.(ii) using the procedure of Example 4.(iii).

6.(iv) Preparation of 3-fluoro-4-methoxybenzyl chloride

The required product was prepared by the procedure described in Example 4.(iv), starting from 3-fluoro-4-methoxybenzyl alcohol prepared in Example 6.(iii).

6.(v) Preparation of $N^1$-(3-fluoro-4-methoxy)benzylpropane-1,2-diamine

To an ice cold solution of 1,2-diaminopropane (3.65 ml) in acetonitrile (50 ml) was added 3-fluoro-4-methoxybenzyl chloride (2.45 g) prepared in Example 6(iv). The reaction mixture was stirred under a nitrogen atmosphere and allowed to attain ambient temperature over 18 hours. A white solid precipitate was removed by filtration. The filtrate was evaporated down to an oil (3.0 g) which was the required product and used with no further purification.

6.(vi) Preparation of 1-(3-fluoro-4-methoxybenzyl)-2-nitroimino-3-methylimidazoline

$N^1$-(3-fluoro-4-methoxy)benzylpropane-1,2-diamine prepared in Example 6(v) and nitroguanidine in water were heated at reflux for 1.5 hours. The reaction mixture was allowed to cool to ambient temperature (20 ° C) and partitioned between water and chloroform (1:1). The chloroform layer was washed with water, then saturated brine, dried over solid magnesium sulphate and evaporated to dryness under reduced pressure. The residue was crystallized from ethyl acetate and the required product obtained as a cream-coloured solid.
m.p. = 105 ° C

| | | C | H | N |
|---|---|---|---|---|
| Analysis: | Theory:<br>Found: | 46.1<br>45.7 | 4.2<br>4.5 | 19.5<br>17.8 |

Further compounds numbered as Examples 7 to 52 in Table 1 were prepared according to procedures analogous to those described above. Comparative Examples, disclosed in other documents, which have been made and tested by the Applicant are noted in Table 2. Referring to Table 2, Comparative Example Number C1 is disclosed in JP 6328776, Comparative Example Number C2 is disclosed in DE OS 25 14 402 A1, Comparative Example Numbers C3 to C6 are disclosed in DE OS 27 32 660 and Comparative Example Numbers C7 to C9 are disclosed in EP 0 136 636. Comparative Example Numbers C10 to C23, made and tested by the Applicant, are noted in Table 3.

**TABLE 1**

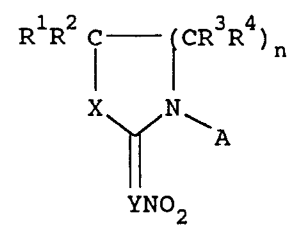

(I)

| EXAMPLE NUMBER | n | $R^1$ | $R^2$ | $R^3$ | $R^4$ | A | X | Y | m.p. (°C) |
|---|---|---|---|---|---|---|---|---|---|
| 1 | 1 | H | H | H | H | 3-fluoro-4-chlorobenzyl | >NH | =CH- | |
| 2 | 2 | H | H | H | H | 3-fluoro-4-chlorobenzyl | >NH | =CH- | 174 |
| 3 | 1 | H | H | H | H | 3-fluoro-4-chlorobenzyl | -S- | =CH- | |
| 4 | 1 | H | H | H | H | 3-fluoro-4-dimethylaminobenzyl | >NH | =CH- | 124 |
| 5 | 2 | H | H | H | H | 3-fluoro-4-dimethylaminobenzyl | >NH | =CH- | 149 |
| 6 | 1 | $CH_3$ | H | H | H | 3-fluoro-4-methoxybenzyl | >NH | =N- | 105 |

**TABLE 1 (Cont.)**

| EXAMPLE NUMBER | n | $R^1$ | $R^2$ | $R^3$ | $R^4$ | A | X | Y | m.p. (°C) |
|---|---|---|---|---|---|---|---|---|---|
| 7 | 2 | H | H | H | H | 3,4-difluorobenzyl | >NH | =CH- | 153 |
| 8 | 2 | H | H | H | H | 2,3-difluorobenzyl | >NH | =CH- | 170 |
| 9 | 2 | H | H | H | H | 3-fluorobenzyl | >NH | =CH- | 209-211 |
| 10 | 1 | H | H | H | H | 3,4-difluorobenzyl | >NH | =CH- | 167 |
| 11 | 1 | H | H | H | H | 3-fluorobenzyl | >NH | =CH- | 150 |
| 12 | 1 | H | H | H | H | 3,5-difluorobenzyl | >NH | =CH- | 169 |
| 13 | 1 | H | H | H | H | 2,3,4-trifluorobenzyl | >NH | =CH- | 196-198 |
| 14 | 1 | H | H | H | H | 3-fluoro-4-methylbenzyl | >NH | =CH- | 153-155 |
| 15 | 2 | H | H | H | H | 3-fluoro-4-methylbenzyl | >NH | =CH- | |
| 16 | 1 | H | H | H | H | 3,6-difluorobenzyl | >NH | =CH- | 136 |
| 17 | 1 | H | H | H | H | 3,4,6-trifluorobenzyl | >NH | =CH- | 200 |
| 18 | 2 | H | H | H | H | 3-fluoro-4-methylthiobenzyl | >NH | =CH- | |
| 19 | 1 | $CH_3$ | H | H | H | 3-fluoro-4-methoxybenzyl | >NH | =CH- | 115 |

**TABLE 1 (Cont.)**

| EXAMPLE NUMBER | n | $R^1$ | $R^2$ | $R^3$ | $R^4$ | A | X | Y | m.p. (°C) |
|---|---|---|---|---|---|---|---|---|---|
| 20 | 1 | H | H | H | H | 3-fluoro-4-diethylaminobenzyl | >NH | =CH- | 175 |
| 21 | 2 | H | H | H | H | 3-fluoro-4-diethylaminobenzyl | >NH | =CH- | 102 |
| 22 | 1 | H | H | H | H | 3-fluoro-4-pyrrolidinobenzyl | >NH | =CH- | 163 |
| 23 | 1 | H | H | H | H | 3-fluoro-4-piperidinobenzyl | >NH | =CH- | 162 |
| 24 | 1 | H | H | H | H | 3-fluoro-4-morpholinobenzyl | >NH | =CH- | 178 |
| 25 | 2 | H | H | H | H | 3-fluoro-4-piperidinobenzyl | >NH | =CH- | 204 |
| 26 | 2 | H | H | H | H | 3-fluoro-4-morpholinobenzyl | >NH | =CH- | 216 |
| 27 | 2 | H | H | H | H | 3-fluoro-4-chlorobenzyl | >NH | =N- | |
| 28 | 1 | H | H | H | H | 3-fluoro-4-chlorobenzyl | >NH | =N- | |
| 29 | 1 | H | H | H | H | 3-fluoro-4-dimethylaminobenzyl | >NH | =N- | 138 |
| 30 | 1 | H | H | H | H | 3-fluoro-4-pyrrolidinobenzyl | >NH | =N- | |
| 31 | 1 | H | H | H | H | 3-fluoro-4-morpholinobenzyl | >NH | =N- | |
| 32 | 1 | H | H | H | H | 3-fluoro-4-piperidinobenzyl | >NH | =N- | |

**TABLE 1 (Cont.)**

| EXAMPLE NUMBER | n | $R^1$ | $R^2$ | $R^3$ | $R^4$ | A | X | Y | m.p. (°C) |
|---|---|---|---|---|---|---|---|---|---|
| 33 | 2 | H | H | H | H | 3-fluoro-4-diethylaminobenzyl | >NH | =N- | |
| 34 | 2 | H | H | H | H | 3-fluoro-4-dimethylaminobenzyl | >NH | =N- | |
| 35 | 1 | H | H | H | H | 3-fluoro-4-diethylaminobenzyl | >NH | =N- | |
| 36 | 2 | H | H | H | H | 3-fluoro-4-morpholinobenzyl | >NH | =N- | |
| 37 | 1 | H | H | H | H | 3-fluoro-4-dimethylaminobenzyl | -S- | =CH- | |
| 38 | 2 | H | H | H | H | 3-fluoro-4-methylaminobenzyl | >NH | =CH- | 154.0 |
| 39 | 1 | H | H | H | H | 3-fluoro-4-methylaminobenzyl | >NH | =CH- | |
| 40 | 1 | H | H | H | H | 3-fluoro-4-methylaminobenzyl | >NH | =N- | |
| 41 | 1 | H | H | H | H | 3-fluoro-4-ethylaminobenzyl | >NH | =CH- | 116.0 |
| 42 | 2 | H | H | H | H | 3-fluoro-4-ethylaminobenzyl | >NH | =CH- | |
| 43 | 2 | H | H | H | H | 3-fluoro-4-ethylaminobenzyl | >NH | =N- | |
| 44 | 1 | H | H | H | H | 3-fluoro-4-ethylaminobenzyl | >NH | =N- | |

**TABLE 1 (Cont.)**

| EXAMPLE NUMBER | n | $R^1$ | $R^2$ | $R^3$ | $R^4$ | A | X | Y | m.p. (°C) |
|---|---|---|---|---|---|---|---|---|---|
| 45 | 1 | H | H | H | H | 3-fluoro-4-isopropylaminobenzyl | >NH | =CH- | |
| 46 | 2 | H | H | 5-$CH_3$ | 5-$CH_3$ | 3-fluoro-4-dimethylaminobenzyl | >NH | =CH- | 128.0 |
| 47 | 1 | H | $CH_3$ | H | H | 3-fluoro-4-dimethylaminobenzyl | >NH | =CH- | 135.0 |
| 48 | 1 | H | $CH_3$ | H | H | 3-fluoro-4-dimethylaminobenzyl | >NH | =N- | |
| 49 | 1 | H | H | H | H | 3,5-difluoro-4-dimethylaminobenzyl | >NH | =N- | 150.0 |
| 50 | 1 | H | H | H | H | 3,6-difluoro-4-dimethylaminobenzyl | >NH | =N- | |
| 51 | 2 | H | H | H | H | 3,6-difluoro-4-dimethylaminobenzyl | >NH | =CH- | |
| 52 | 1 | H | H | H | H | 3,6-difluoro-4-dimethylaminobenzyl | >NH | =CH- | |

TABLE 2

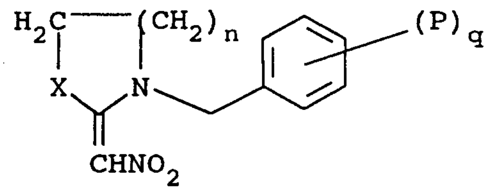

| EXAMPLE NUMBER | n | q | P | X |
|---|---|---|---|---|
| C1 | 2 | 1 | 3-cyano | >NH |
| C2 | 2 | 0 | - | >NH |
| C3 | 1 | 1 | 4-fluoro | >NH |
| C4 | 1 | 1 | 2-chloro | >NH |
| C5 | 1 | 1 | 4-chloro | >NH |
| C6 | 1 | 2 | 3,4-dichloro | >NH |
| C7 | 2 | 1 | 4-chloro | >NH |
| C8 | 2 | 1 | 4-fluoro | >NH |
| C9 | 2 | 1 | 4-bromo | >NH |

**TABLE 3**

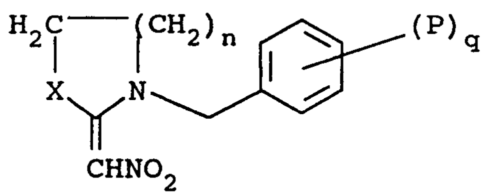

| EXAMPLE NUMBER | n | q | P | X |
|---|---|---|---|---|
| C10 | 2 | 1 | 4-chloro | >NH |
| C11 | 2 | 0 | - | >NH |
| C12 | 1 | 1 | 4-fluoro | >NH |
| C13 | 2 | 1 | 3-bromo | >NH |
| C14 | 1 | 1 | 4-chloro | >NH |
| C15 | 1 | 2 | 3,4-dichloro | >NH |
| C16 | 1 | 2 | 3-bromo-4-methyl | >NH |
| C17 | 1 | 2 | 3-bromo-4-methoxy | >NH |
| C18 | 2 | 2 | 3-bromo-4-methyl | >NH |
| C19 | 1 | 2 | 3-chloro-4-fluoro | >NH |
| C20 | 2 | 2 | 3-chloro-4-fluoro | >NH |
| C21 | 1 | 2 | 3-chloro-4-fluoro | >NH |
| C22 | 2 | 2 | 3-chloro-4-methyl | >NH |
| C23 | 1 | 2 | 3-chloro-4-fluoro | -S- |

**EXAMPLE 53**

**Pesticidal Activity**

Pesticidal activity of compounds of the invention was assessed against various of the following pests:-
Spodoptera littoralis (Egyptian cotton leafworm)
Aedes aegypti (yellow fever mosquito)
Musca domestica (housefly)
Acyrthosiphon pisum (pea aphid)
Megoura viciae (vetch aphid)
Phaedon cochleariae (mustard beetle)
Trialeurodes vaporariorum (greenhouse whitefly)

Nephotettix cincticeps (green leaf hopper)
Nilaparvata lugens (brown rice plant hopper)

The test methods employed for each species appear below. In each test, unless otherwise stated, solutions or suspensions of test compound were made up over a range of concentrations in water (initially 0.1%w) containing 10%w acetone and 0.025%w "TRITON X-100" (trade mark) surface active agent (the condensation product of ethylene oxide with an alkyl phenol). These solutions were sprayed at a rate equivalent to 340 litres per hectare (3.4 x $10^{-5} m^3/m^2$) onto Petri dishes containing either test species per se or diet onto which test species were subsequently introduced, as indicated. In some assays leaf discs infested with test species were sprayed whilst other assays involved the spraying of plants which were infested subsequently with test species after the spray solution had dried. The tests were all conducted under normal insectary conditions (23°C ± 2°C, fluctuating humidity and light). Mortality assessments were made as indicated below, in terms of percentage mortality figures. In each test a $LC_{50}$ (the dosage of active material required to kill half of the test species) for the compound was calculated from the mortality figures and compared with the corresponding $LC_{50}$ for a standard insecticide, ethyl parathion, in the same test. The results are expressed as toxicity indices thus:

$$\text{toxicity index} = \frac{LC_{50}\ \text{(parathion)}}{LC_{50}\ \text{(test compound)}} \times 100$$

(i) Spodoptera littoralis (Diet)

Test solutions were sprayed as indicated above onto Petri dishes containing a nutritious diet for Egyptian cotton leafworm larvae. When the spray deposit had dried, each dish was infested with ten 2nd instar larvae. Mortality assessments were made 1 day (Sl-D-1d) and 7 days (Sl-D-7d) after spraying.

(ii) Spodoptera littoralis (foliar) (Sl Fol)

Test solutions were sprayed as described above onto Petri dishes containing 9cm discs of Chinese cabbage leaves on filter papers. After drying, each dish was infested with ten 2nd instar larvae. Mortality assessments were made 24 hours after infestation.

(iii) Spodoptera littoralis (ovicidal) (S1 OA)

Test solutions were sprayed as described above onto Petri dishes containg filter papers on which were approximately 50 24 hour old eggs. After 6 days the numbers of hatched and unhatched eggs were counted and percentage mortality calculated.

(iv) Aedes aegypti (Aa)

Early 4th instar larvae were used. Test solutions were made up to 0.5 ppm of test compound (and progressive half-dilutions) in water containing 0.04%w "TRITON X-100" (trade mark); acetone was initially present to aid solution, but was allowed to evaporate off before introduction of larvae.

Ten early 4th instar larvae were placed in 100 ml of test solution held at 28°C, and after 48 hours, larval mortality was recorded (Aa-2d). The final mortality was assessed by counting the number of emerged adult mosquitoes after one weed (Aa-7d).

(v) Musca domestica (Md)

Batches of ten 2 to 3 day old milk-fed adult female houseflies, anaesthetised using carbon dioxide, were placed on filter papers inside Petri dishes. The dishes were sprayed with the test solutions as described above. The flies were retained in the Petri dishes and were fed with a dilute milk solution which was dripped down the side of the Petri dish and absorbed by the filter paper. Mortality was assessed after 24 hours.

(vi) Acyrthosiphon pisum (Ap)

Tests were carried out on young adult pea aphids. Whole pea plants 6 days after germination were placed on filter papers in Petri dishes. Ten aphids were transferred to each pea plant and left for 30 minutes to allow the aphids to settle and start to feed. The dishes were then sprayed with the test solutions as described above and lids were placed on the Petri dishes. Mortality was assessed after 24 hours.

(vii) Megoura viciae (Mv)

Tests were carried out on adult-Vetch aphids. Pairs of broad bean leaves on filter paper in Petri dishes were sprayed side by side with uncounted quantities of aphids in small gauze-covered containers. After passing through the spray ten aphids were tipped onto the leaves and lids were placed on the Petri dishes. Mortality was assessed after 24 hours.

(viii) Phaedon cochleariae (Pc)

Test solutions were sprayed as described above onto Petri dishes containing 9cm discs of Chinese cabbage leaves on filter papers. After drying, each dish was infested with ten adult mustard beetles (up to 1 week old). Mortality assessments were made 24 hours after infestation.

(ix) Trialeurodes vaporariorum (Tv)

French bean plants (Phaseolus vulgaris) with two fully expanded leaves were placed in a breeding culture of T. vaporariorum, also on French bean plants, which were then disturbed to ensure resettlement on the introduced plants. During the subsequent 24 hour period, eggs were deposited and kept at 27°C, with 14 hours photoperiod. All adult whiteflies were then carefully removed, leaving egg samples of a known age. After eight days the majority of eggs had hatched. Leaf discs containing the newly hatched nymphs were then cut from the leaves and transferred to moist filter paper. The discs were examined under a low-powered microscope to determine the exact number of 1st instar nymphs per disc and to remove any unhatched eggs. On average, 70-100 nymphs were found per disc.

The discs were transferred into Petri dishes and sprayed with test solutions as described above. After 6 days percentage mortalities were assessed.

(x) Nephotettix cincticeps (Nc)

Tests wee carried out on young adult female green leaf hoppers. Plant pots, each containing five rice seedlings 10 to 15 cm tall arranged across the centre of the pot, were sprayed with test solutions as described above (but initial test concentration 0.05% of test compound). Spraying was on both sides of the plants with the post horizontal. One hour after spraying, each pot was filled to the brim with fine silver sand, an open-ended glass jar was placed over each pot and each pot was infested with ten hoppers. A paper tissue was placed over the open end of each glass jar to retain the hoppers. The pots were irrigated from underneath, maintained at a temperature of 27°C ± 2°C and subjected to white fluorescent light under a regime of 18 hours light followed by 6 hours darkness. Mortality assessments were made 48 hours after infestation.

(xi) Megoura viciae systemic [Mv(syst)] and spodoptera littoralis systemic [Sl(syst)]

Tests were carried out on young apterous adults of the vetch aphid Megoura viciae and 3rd instar larvae of leaf fed Spodoptera littoralis.

The test solution comprised the test compound dissolved in aqueous acetone (5%) containing Tween 20 (Trade Mark) (0.05%) as a wetting agent. The standard for the tests was Metasystox (Trade mark) applied over the range 0.0001% - 0.00001%.

Broad bean plants with three fully expanded leaf pairs were selected. The second leaf pair from the base of the stem on each plant was trimmed with scissors to produce a 4 cm cut edge on each side and a 2 cm cut end. The prepared leaves were promptly immersed in 10 ml of test solution contained in a small glass beaker, which was then covered with a layer of Parafilm (Trade Mark). The beaker was held on a support stand next to, and at the same height as, the cut leaf. After 48 hours immersion, the treated leaves were cut from the rest of the plant. New growth leaves which expanded after treatment were used for the

following bioassay.

Small perspex ring cages of internal diameter 20 mm containing 10 aphids were held onto the underside of one leaf from the leaf pair by means of spring clips. Mortality assessments of the aphids were made after 24 hours. The other leaf of the pair was then cut off and placed in a 9 cm diameter petri-dish containing ten Spodoptera littoralis larvae. Mortality assessments were made after 24 hours.

The results are shown in Table 4 using the grading system shown below, where % concentration refers to the solution used for the leaf immersion.

| Grade | Range (% concentration) | | |
|---|---|---|---|
| 0 | no activity at 0.1% | | |
| 1 | 0.1 | > LC50 | > 0.03 |
| 2 | 0.03 | > LC50 | > 0.01 |
| 3 | 0.01 | > LC50 | > 0.03 |
| 4 | 0.003 | > LC50 | > 0.001 |
| 5 | 0.001 | > LC50 | > 0.0003 |
| 6 | 0.0003 | > LC50 | > 0.0001 |
| 7 | 0.0001 | > LC50 | > 0.00003 |
| 8 | 0.00003 | > LC50 | > 0.00001 |

**EXAMPLE 54**

**Acaricidal Activity (ovicide) (Tu OA)**

Acaricidal activity of the compounds of the invention was assessed, employing eggs of the glasshouse red spider mite, Tetranychus urticae (T.u.), less than 24 hours old, by the following procedure.

2cm diameter leaf discs cut from the leaves of French bean plants were placed on filter paper, kept moist by a cotton wool wick dipped into water.

On the day before spraying, each leaf disc was infested with 10 female adult mites. On the day of the test, the adults were removed, leaving the eggs laid overnight on the discs. The leaf discs were then sprayed with solutions of test compound made up as in Example 53 above, at a rate equivalent to 340 litres per hectare ($3.4 \times 10^{-5}$ $m^3/m^2$).

Throughout the test, the eggs were held under normal insectary conditions (23°C ± 2°C),fluctuating humidity and 16 hours days length). After 7-10 days, the numbers of hatched nymphs and unhatched eggs were assessed and the percentage mortality calculated. The $LC_{50}$ (the dosage of active material required to kill half of the test species) for the compound was calculated from the mortality figure and compared with the corresponding $LC_{50}$ for a standard insecticide, chlorfenson, in the same test. The result is expressed as toxicity index thus:

$$\text{toxicity index} = \frac{LC_{50}\ (\text{chlorfenson})}{LC_{50}\ (\text{test compound})} \times 100$$

Table 4 provides the results for the tests of Examples 53 and 54. The method of expression of the results for Example 53(xi) is described in the write-up for that Example. For the other Examples grades A, B and C indicate mortalities of 70-100%, 40-69% and 0-39%, respectively, at the initial test concentration of 0.1% (1000 ppm).

For most compounds which scored a grade A at 0.1% (1000 ppm), the Toxicity Index (TI) was calculated as described above.

In Table 4, the TI values are reported as follows:

TI < 10 = X

TI 10-100 = XX

TI > 100 = XXX
"-" means no data available.

TABLE 4

| Compound of Eg. No. | Sl-fol | Sl-D 1d | Sl-D 7d | Aa-2d | Aa-7d | Md | Ap | Tu OA | Sl-OA | Nc | Mv Syst. | Sl Syst. | Tv | Pc |
|---|---|---|---|---|---|---|---|---|---|---|---|---|---|---|
| 1 | B | C | C | C | X | C | XXX | C | XX | XXX | 1 | 0 | XX | X |
| 2 | C | C | C | C | X | C | XXX | C | XX | XX | 2 | 0 | B | X |
| 3 | C | C | C | - | C | C | C | C | C | XX | 0 | 0 | C | X |
| 4 | XXX | XX | XX | C | X | C | XX | C | - | XXX | 5 | 3 | - | - |
| 5 | XX | C | C | - | C | C | C | C | - | XXX | 2.5 | 1 | - | - |
| 6 | C | C | C | - | C | C | A | C | - | C | 0 | 0 | - | - |
| 7 | C | C | C | - | C | C | C | C | C | XX | 1 | 0 | B | - |
| 8 | C | C | C | C | C | C | C | XX | XX | C | 0 | 0 | C | C |
| 9 | C | C | C | C | X | C | X | C | C | XX | 2 | 0 | C | - |
| 10 | C | C | C | C | X | C | X | C | XX | XX | 1 | 0 | C | C |
| 11 | C | C | C | C | X | C | X | C | C | XX | 1.5 | 0 | C | B |
| 12 | C | C | C | - | C | C | C | C | XX | C | 0 | 0 | C | C |
| 13 | C | C | C | - | C | C | C | C | C | B | 0 | 0 | C | C |
| 14 | C | C | C | - | C | C | XX | C | X | XX | 0 | 0 | B | C |
| 15 | C | C | C | - | C | C | XX | C | - | XX | 1 | 0 | C | X |

**TABLE 4 (Cont.)**

| Compound of Eg. No. | Sl-fol | Sl-D 1d | Sl-D 7d | Aa-2d | Aa-7d | Md | Ap | Tu OA | Sl-OA | Nc | Mv Syst. | Sl Syst. | Tv | Pc |
|---|---|---|---|---|---|---|---|---|---|---|---|---|---|---|
| 16 | C | C | C | - | C | C | C | C | - | B | 0 | 0 | - | - |
| 17 | C | C | C | - | C | C | C | C | C | XX | 0 | 0 | C | - |
| 18 | C | C | C | - | C | C | C | C | - | C | 0 | 0 | - | - |
| 19 | C | C | C | - | C | C | X | C | - | C | 0 | 0 | - | - |
| 20 | - | C | A | C | A | C | A | C | C | A | - | - | - | - |
| 21 | - | - | - | - | - | - | - | - | - | - | - | - | - | - |
| 22 | C | C | C | - | C | C | C | C | - | C | 0 | 0 | - | - |
| 23 | C | C | C | - | C | C | C | C | - | C | 0 | 0 | - | - |
| 24 | C | C | C | - | C | C | C | C | - | C | 0 | 0 | - | - |
| 25 | C | C | C | C | C | C | C | C | - | C | 0 | 0 | - | - |
| 26 | C | C | C | - | C | C | C | C | - | C | 0 | 0 | - | - |
| 27 | C | C | C | - | C | C | C | C | C | C | 0 | 0 | B | C |
| 28 | C | C | C | - | C | C | C | C | C | C | 0 | 0 | B | C |
| 29 | C | C | C | - | C | C | C | C | - | C | 2.5 | 0 | - | - |
| 30 | - | C | C | - | C | C | C | C | - | C | 0 | 0 | - | - |

**TABLE 4 (Cont.)**

| Compound of Eg. No. | Sl-fol | Sl-D 1d | Sl-D 7d | Aa-2d | Aa-7d | Md | Ap | Tu OA | Sl-OA | Nc | Mv Syst. | Sl Syst. | Tv | Pc |
|---|---|---|---|---|---|---|---|---|---|---|---|---|---|---|
| 31 | C | C | C | - | C | C | C | C | - | C | 0 | 0 | - | - |
| 32 | - | - | - | - | - | - | - | - | - | - | - | - | - | - |
| 33 | - | C | C | C | C | C | C | A | C | - | - | - | - | - |
| 34 | C | C | C | - | C | C | C | C | - | C | 0 | 0 | - | - |
| 35 | C | C | C | - | C | C | C | C | - | C | 0 | 0 | - | - |
| 36 | C | C | C | - | C | C | C | C | - | C | 0 | 0 | - | - |
| 37 | C | C | C | - | C | C | C | C | - | - | - | - | - | - |
| 38 | B | C | C | C | A | C | A | C | - | - | - | - | - | - |
| 39 | C | C | C | - | C | C | C | C | - | B | <3 | 1 | C | - |
| 40 | C | C | C | - | C | C | C | C | - | C | 0 | 0 | - | - |
| 41 | A | C | C | C | C | C | C | C | - | XXX | 0 | 2.5 | C | - |
| 42 | C | C | C | C | C | C | C | C | - | XXX | 2 | 1.5 | C | - |
| 43 | C | C | C | - | C | C | C | C | - | C | 0 | 0 | - | - |
| 44 | C | C | C | - | C | C | C | C | - | - | 0 | 0 | - | - |
| 45 | A | C | C | - | C | C | C | C | - | C | 0 | A | - | - |
| 46 | C | C | C | - | C | C | C | C | - | C | 0 | 0 | - | - |

**TABLE 4 (Cont.)**

| Compound of Eg. No. | Sl-fol | Sl-D 1d | Sl-D 7d | Aa-2d | Aa-7d | Md | Ap | Tu OA | Sl-OA | Nc | Mv Syst. | Sl Syst. | Tv | Pc |
|---|---|---|---|---|---|---|---|---|---|---|---|---|---|---|
| 47 | B | C | C | - | C | C | C | C | - | XXX | 3 | 0 | - | - |
| 48 | C | C | C | - | C | C | C | C | - | C | 0 | 0 | - | - |
| 49 | C | C | C | - | C | C | C | C | - | C | 3.5 | 0 | - | - |
| 50 | C | C | C | - | C | C | C | C | - | C | 0 | 0 | - | - |
| 51 | C | C | C | - | C | C | C | C | - | XX | 0 | 0 | - | - |
| 52 | B | C | C | - | C | C | C | C | - | C | 0 | 0 | - | - |
| C1 | C | C | C | - | C | C | X | C | C | XXX | 0 | 0 | B | C |
| C2 | - | C | C | C | C | C | - | C | B | B | - | - | - | - |
| C3 | - | C | C | C | C | C | X | C | XX | B | - | - | - | - |
| C4 | - | C | C | C | C | C | C | C | C | C | - | - | - | - |
| C5 | - | C | C | X | X | C | XX | C | XX | XX | 0 | 1.5 | - | - |
| C6 | C | C | C | - | C | C | C | C | - | C | 0 | 0 | C | - |
| C7 | - | C | C | C | X | C | - | C | C | XX | 2 | 0 | - | - |
| C8 | - | C | C | C | C | C | X | C | C | B | - | - | - | - |
| C9 | - | C | C | C | C | C | C | C | C | XX | - | - | - | - |

**TABLE 4 (Cont.)**

| Compound of Eg. No. | Sl-fol | Sl-D 1d | Sl-D 7d | Aa-2d | Aa-7d | Md | Ap | Tu OA | Sl-OA | Nc | Mv Syst. | Sl Syst. | Tv | Pc |
|---|---|---|---|---|---|---|---|---|---|---|---|---|---|---|
| C10 | - | C | C | C | X | C | - | C | C | XX | 2 | 0 | - | - |
| C11 | - | C | C | C | C | C | - | C | B | B | - | - | - | - |
| C12 | - | C | C | C | C | C | X | C | XX | B | - | - | - | - |
| C13 | - | C | C | C | C | C | C | C | C | C | - | - | - | - |
| C14 | - | C | C | X | X | C | XX | C | XX | XX | 0 | 1.5 | - | - |
| C15 | C | C | C | - | C | C | C | C | - | C | 0 | 0 | C | - |
| C16 | C | C | C | - | C | C | C | C | C | C | 0 | 0 | C | C |
| C17 | C | C | C | - | C | C | C | C | C | C | 0 | 0 | C | C |
| C18 | C | C | C | - | C | C | C | C | C | C | 0 | 0 | C | C |
| C19 | C | C | C | - | C | C | X | C | - | C | 0 | 0 | - | - |
| C20 | C | C | C | - | C | C | X | C | - | - | 0 | 0 | - | - |
| C21 | C | C | C | - | C | C | C | C | - | C | 0 | 0 | - | - |
| C22 | C | C | C | - | C | C | C | C | - | C | 0 | 0 | - | - |
| C23 | C | C | C | - | C | C | C | C | - | C | 0 | 0 | - | - |

## Claims

**1.** A compound of general formula:

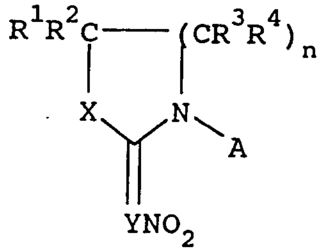

(I)

in which n is 1, 2 or 3; $R^1$, $R^2$, $R^3$ and $R^4$ independently represent a hydrogen atom or an alkyl group; X represents an oxygen atom or a group $>CR^5R^6$, $>NR^7$, $>NCOR^8$ or $>S(O)_p$, where $R^5$, $R^6$, $R^7$ and $R^8$ independently represent a hydrogen atom or an alkyl group and p is 0, 1 or 2; A represents an optionally substituted 3-fluorobenzyl group; and Y represents a group =CH- or =N-.

**2.** A compound as claimed in claim 1, wherein n is 1 or 2.

**3.** A compound as claimed in any preceding claim, wherein each of $R^1$, $R^2$, $R^3$ and $R^4$ represents a hydrogen atom or a methyl group.

**4.** A compound as claimed in any preceding claim, wherein X represents a group $>NR^7$.

**5.** A compound as claimed in any preceding claim, wherein optional substituents of said 3-fluorobenzyl group are selected from halogen atoms, or alkyl or alkylamino groups.

**6.** A compound as claimed in any preceding claim, wherein an optional substituent of said 3-fluorobenzyl group is a dimethylamino or diethylamino group.

**7.** A compound as claimed in any preceding claim, wherein said 3-fluorobenzyl group is substituted at the 4-position.

**8.** A process for the preparation of a compound of general formaul I, the process comprising:

(a) reacting a compound of general formula

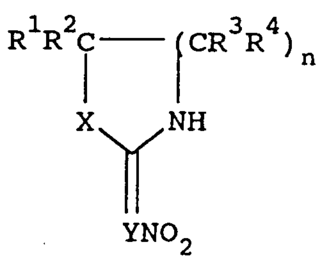

(II)

with a compound of general formula $AL^1$ or a salt thereof, wherein n, $R^1$, $R^2$, $R^3$, $R^4$, X, A and Y are as defined in any of claims 1 to 7 and $L^1$ is a leaving group, and optionally derivatising the product of the reaction in order to prepare a compound of general formula I; or

(b) reacting a compound of general formula

$HX\text{-}CR^1R^2\text{-}(CR^3R^4)_n\text{-}NH\text{-}A$ (III)

with 1,1-bis(methylthio)-2-nitroethylene or with nitroguanidine where n, $R^1$, $R^2$, $R^3$, $R^4$, X and A are as defined in any of claims 1 to 7, and optionally derivatising the product of the reaction in order to prepare a compound of general formula I.

9. A compound of general formula I as defined in any of claims 1 to 7, when prepared by a process as defined in claim 8.

10. A pesticidal composition comprising a compound of general formula I as claimed in any of claims 1 to 7 or 9 together with a carrier.

11. A method of combating pests at a locus which comprises applying to the locus a pesticidal compound as claimed in any of claims 1 to 7 or 9 or a composition as claimed in claim 10.

12. Use of a compound of general formula I as defined in any of claims 1 to 7 or 9 as a pesticide.

13. A compound of general formula I, a process for the preparation of a compound of general formula I, a compound of general formula I prepared by the process, a pesticidal composition including a compound of general formula I, a method of combatting pests and the use of a compound of general formula I, each being substantially as hereinbefore described, with reference to the examples.

European Patent Office

# EUROPEAN SEARCH REPORT

Application Number
EP 94 20 1160

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,X | DE-A-27 32 660 (HOECHST)<br>* claims 1,4 * | 1,10 | C07D233/20<br>C07D233/52<br>C07D239/06<br>C07D239/18<br>C07D277/10<br>C07D277/18<br>C07D207/20<br>C07D207/22<br>A01N43/50<br>A01N43/54<br>A01N43/36<br>A01N43/78 |
| D,X | EP-A-0 136 636 (NIHON TOKUSHU NOYAKU SEIZO)<br>* claims 1,6 * | 1,10 | |
| X | EP-A-0 199 974 (NIHON TOKUSHU NOYAKU SEIZO)<br>* claims 1,6 * | 1,10 | |
| X | GB-A-1 508 880 (SYNTHELABO)<br>* page 15; claim 17 * | 1 | |

TECHNICAL FIELDS SEARCHED (Int.Cl.5)
C07D
A01N

The present search report has been drawn up for all claims

| Place of search | Date of completion of the search | Examiner |
|---|---|---|
| THE HAGUE | 9 June 1994 | Voyiazoglou, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document